(19) Europäisches Patentamt / European Patent Office / Office européen des brevets

(11) **EP 1 787 970 A1**

(12) **DEMANDE DE BREVET EUROPEEN**

(43) Date de publication:
**23.05.2007 Bulletin 2007/21**

(21) Numéro de dépôt: **07004829.3**

(22) Date de dépôt: **10.08.2001**

(51) Int Cl.:
*C07C 37/74* (2006.01)    *A23G 1/00* (2006.01)
*A23G 1/04* (2006.01)    *A23L 3/3472* (2006.01)
*A23L 1/30* (2006.01)    *C07C 39/00* (2006.01)

(84) Etats contractants désignés:
**AT BE CH CY DE DK ES FI FR GB GR IE IT LI LU MC NL PT SE TR**

(30) Priorité: **11.08.2000 FR 0010603**

(62) Numéro(s) de document de la (des) demande(s) initiale(s) en application de l'article 76 CBE:
**01963095.3 / 1 309 532**

(71) Demandeur: **Barry Callebaut AG**
**8008 Zurich (CH)**

(72) Inventeurs:
• **Lecoupeau, Jean-Paul**
**27940 Venables (FR)**
• **Vercauteren, Joseph**
**34170 Castelnau-le-Lez (FR)**

(74) Mandataire: **Tarrère, Blandine et al**
**Cabinet Armengaud Aîné**
**3, Avenue Bugeaud**
**75116 Paris (FR)**

Remarques:
Cette demande a été déposée le 08 - 03 - 2007 comme demande divisionnaire de la demande mentionnée sous le code INID 62.

(54) **Procede d'obtention d'extraits polyphenoliques de feves de cacao , les extraits obtenus et leurs applications**

(57)    L'invention se rapporte à un procédé d'obtention d'extraits à base de composés polyphénoliques contenus dans le cacao, caractérisé en ce qu'il comprend:
- la mise en oeuvre de fèves fraîches, n'ayant pas subi de pré-traitement, ni de dégraissage, ces fèves étant débarrassées de leurs pulpe et coque, de manière à obtenir des amandes propres,
- le concassage desdites amandes, en présence de solvant(s),
- la macération des amandes concassées dans des conditions permettant d'extraire les composés recherchés,
- la filtration du mélange de macération,
- la récupération d'un extrait renfermant lesdits composés à partir du filtrat,
    L'invention vise également les extraits et leurs applications en cosmétique, alimentaire et thérapeutique.

Fig. 1

EP 1 787 970 A1

## Description

La présente invention concerne un procédé d'obtention d'extraits à base de composés polyphénoliques à partir de fèves de cacao, les extraits obtenus et leurs applications.

On sait que la graine fraîche de cacao contient environ 40% d'eau, 30 à 35% de lipides, 4 à 6% de polyphénols ou de dérivés de polyphénols, 1,5% de xanthines, le reste étant essentiellement constitué de protéines, d'amidon, de cellulose et de sucres. A cet égard, on peut se reporter notamment aux articles suivants :

- « Cacao procyanidins : major flavanoids and identification of some minor metabolites » de L.J. Porter, Z. Ma et B.G. Chan, publié dans Phytochemistry vol. 35, n° 5 p1657-1663, 1991 et

- Epicatechin content in fermented and unfermented cocao beans » de H. Kim et P.G. Keeney, publié dans Journal of Food Science - vol. 49 (1984) p 1090-1092.

On notera que le terme « polyphénol », tel qu'utilisé dans la description et les revendications désigne les polyphénols non substitués, et substitués notamment sous forme de glycosides. Ces polyphénols appartiennent en particulier à la classe des anthocyanines, des flavonoïdes et, des flavanols et leurs oligomères, de types A et/ou B.

De même, on notera que le terme lipide ou matière grasse représente des acides gras libres, des stérols, en particulier des phytostérols, des mono, di et triglycérides.

Par pré-traitement, on entend une opération de fermentation et/ou séchage et/ou lavage.

On sait que le cacao est originaire d'Amérique du Sud et que ses fruits ou cabosses sont cueillis, les fèves subissent un pré-traitement qui consiste à fermenter pendant cinq à six jours avant d'être séchées. Au cours de cette fermentation, il se produit un certain nombre de réactions biochimiques se traduisant, notamment, par la destruction de micro-organismes pathogènes, la formation de précurseurs d'arômes et une dégradation partielle des polyphénols par suite d'une oxydation enzymatique ou d'un tannage des protéines. On considère que 70 à 80% des polyphénols sont dégradés lors de la fermentation.

Or, les polyphénols sont les substances naturelles anti-oxydantes et anti-radicalaires les plus puissantes que l'on connaisse. Les extraits polyphénoliques et les préparations qui en contiennent sont classiquement utilisés dans les indications suivantes : troubles circulatoires, insuffisances veino-lymphatiques, fragilité capillaire cutanée, troubles circulatoires de la rétine, crise hémorroïdaire, érythèmes solaires ou liés à l'action de radiations (prévention des dégâts causés par la radiothérapie), hypertension, hypercholestérolémie , diverses affections virales et microbiennes.

Ces dernières années, de nombreuses publications ont révélé au niveau moléculaire les modes d'action par lesquels ils sont capables de lutter contre les affections majeures que sont :

- les maladies cardiovasculaires :

  - Antiagrégants plaquettaires (Petroni, A., M. Blasevich, M. Salami, N. Papini, G.F. Montedoro and C. Galli, Inhibition of platelet aggregation and eicosanoid production by phenolic components of olive oil. Thromb Res, 1995. 78(2): p. 151-160)
  - Anti-inflammatoires et protecteur de l'oxydation des LDL-cholestérol (Frankel, E., J. Kanner, J. German, E. Parks and J. Kinsella, Inhibition of oxidation of human low-density lipoprotein by phenolic substances in red wine. Lancet, 1993. 341(8843): p. 454-457)
  - Protecteur de l'oxydation des éicosanoïdes (Pace-Asciak, C.R., S. Hahn, E.P. Diamandis, G. Soleas and D.M. Goldberg, The red wine phenolics trans-resveratrol and quercetin block human platelet aggregation and eicosanoid synthesis: implications for protection against coronary heart disease. Clin Chim Acta, 1995. 235(2): p. 207-219)
  - Anti-athérosciérotiques (Yamakoshi, J., S. Kataoka, T. Koga and T. Ariga, Proanthocyanidin-rich extract from grape seeds attenuates the development of aortic atherosclerosis in cholesterol-fed rabbits. Atherosclerosis, 1999. 142(1): p. 139-149)
  - Anti-thrombotiques (Fuhrman, B., A. Lavy and M. Aviram, Consumption of red wine with meals reduces the susceptibility of human plasma and low-density lipoprotein to lipid peroxidation. Am J Clin Nutr, 1995. 61(3): p. 549-554)

- l'Alzheimer (Orgogozo, J.M., J.F. Dartigues, S. Lafont, L. Letenneur, D. Commenges, R. Salamon, S. Renaud and M. Breteler, Wine consumption and dementia in the elderly: A prospective community study in the Bordeaux area. Rev Neurol, 1997. 153(3): p. 185-192)

- le cancer (Jang, M.S., E.N. Cai, G.O. Udeani, K.V. Slowing, C.F. Thomas, C.W.W. Beecher, H.H.S. Fong, N.R. Farnsworth, A.D. Kinghorn, R.G. Mehta, R.C. Moon and J.M. Pezzuto, Cancer chemopreventive activity of resveratrol, a natural product derived from grapes. Science, 1997. 275(5297): p. 218-220)

Compte tenu du fait que le cacao contient des polyphénols et de l'importance de l'utilisation des polyphénols dans le domaine médical, on a été amené à tenter d'extraire du cacao, les composés polyphénoliques qu'il contient notamment dans le but de réaliser des aliments et boissons diététiques contenant cet anti-oxydant. Un pré-traitement comportant une fermentation suivie d'une opération de séchage constitue un inconvénient majeur en ce sens qu'il nuit au rendement de l'extraction des polyphénols contenus dans le cacao.

En cherchant à pallier ces inconvénients, les inventeurs ont découvert que l'utilisation de fèves n'ayant pas subi de traitement préalable et la réalisation de l'extraction dans des conditions déterminées permettrait d'obtenir des extraits de composition originale, dotés de propriétés de grand intérêt.

L'invention a donc pour but de fournir un procédé d'extraction de fèves de cacao permettant de disposer d'extraits à forte teneur en polyphénols et enrichis, (par rapport aux teneurs initiales dans les fèves) en certains dérivés lipidiques d'intérêt. Elle vise également à fournir de tels extraits en tant que produits nouveaux.

L'invention vise en outre la mise à profit des propriétés ou des extraits dans diverses applications, notamment dans le domaine alimentaire, cosmétique et thérapeutique.

Le procédé, selon l'invention, d'obtention d'extraits à base de composés polyphénoliques, à partir de fèves de cacao, est caractérisé en ce qu'il comprend

- la mise en oeuvre de fèves fraîches, n'ayant pas subi de pré-traitement, ni de dégraissage, ces fèves étant débarrassées de leurs pulpe et coque, de manière à obtenir des amandes propres,
- le concassage desdites amandes, en présence de solvant,
- la macération des amandes concassées dans des conditions permettant d'extraire les composés recherchés,
- la filtration du mélange de macération,
- la récupération d'extrait renfermant lesdits composés à partir du filtrat.

Les fèves utilisées sont pré-traitées ou non.

Selon une variante de la présente invention, on met en oeuvre des fèves de cacao marchandes, c'est-à-dire des fèves ayant subi un pré-traitement comportant un séchage, dont les grains sont réhumidifiés avant concassage, par exemple avec 30 à 50% d'eau tiède.

L'étape de macération est réalisée à l'aide d'eau ou d'un mélange d'eau et de plusieurs solvants, capables de solubiliser les polyphénols et les lipides, sans altérer leurs propriétés, tels que l'éthanol, l'acétone, le butanol 2, le propanol 2. De préférence, la teneur en solvant est supérieure à 50% en volume.

On opère avantageusement à une température, de l'ordre de 20 à 50°C, pendant 1 heure à plusieurs jours.

Comme le montrent les exemples, une macération de seulement 1 h, avec un solvant tel que l'éthanol 70° permet d'obtenir des extraits de grande qualité, ce qui présente un grand intérêt pour les applications industrielles du procédé.

Le mélange obtenu est alors filtré et le filtrat traité pour récupérer l'extrait recherché.

De manière avantageuse, le gâteau de filtration est au préalable soumis à une ou plusieurs étapes dé lavage. On utilise en particulier le même solvant que celui mis en oeuvre dans l'étape de macération.

La récupération de l'extrait à base de composés polyphénoliques consiste en particulier en une distillation, conduite de manière à évaporer le solvant et obtenir un extrait.

Les extraits obtenus présentent l'avantage d'une teneur élevée en composés polyphénoliques et un enrichissement, par rapport à la composition des fèves de départ, en phytostérols, notamment en β-sitostérol.

De tels extraits constituent des produits nouveaux et, à ce titre, entrent également dans le champ de l'invention.

L'invention vise en particulier des extraits caractérisés par une teneur (% en poids par rapport à l'extrait total) de 15 à 65% en polyphénols, de 0 à 11% en lipides et de 0 à 20% en xanthines.

L'invention vise en particulier les extraits dans lesquels les lipides comportent de 10 à 30% en poids de phytostérols avec de préférence, 7 à 15% de β-sitostérol. Ce taux préférentiel est atteint lorsque le procédé selon l'invention est mis en oeuvre sur les fèves fraîches.

De tels extraits originaux constituent avantageusement une signature du procédé de l'invention.

On a déjà indiqué en début de description les propriétés mises en évidence chez les polyphénols. De même, les phystostérols constituent des produits de grand intérêt.

Les phytostérols oxydés sont des phytonutriments dont les qualités nutritionnelles en santé publique sont particulièrement bien documentées. De nombreux travaux scientifiques ont montré le rôle, en particulier, du β-sitostérol dans la protection et la prévention contre certaines maladies.

On peut résumer ainsi ces qualités :

- un effet stimulant sur le système immunitaire en augmentant les défenses immunologiques contre des infections virales et bactériennes (Bouic, P.J.D. et al. International Journal of Immunopharmacology, vol. 18, no. 12, p. 693-700, Dec. 1996),

- un effet hypocholestérolémiant chez l'homme sans changement de diète alimentaire ni de modification de l'activité physique (Métab. Clin. Exper., vol. 38, p. 136-40 (1989) ; American J. Clin. Nutr., vol. 59, p. 1325-31 (1994)),

- combattre les désordres liés au stress (immunosuppression, douleurs et névralgies...), P.J.D. Bouic et al: International Journal of Sports Medicine, 1999,

- combattre les affections de la prostate, Klippel K. F. et al: British Journal of Urology, v. 80 (3), pp. 427-432, Sept. 1997,

- combattre le cancer de la prostate et du sein,

- combattre certaines maladies auto-immune comme le Lupus, le Psoriasis, le Syndrome de fatigue chronique ainsi que la polyarthrite rhumatoïde, (P.J.D. Bouic : Newsletter of the Arthritis Trust of America, Summer 1998),

- maintenir un certain taux de lymphocytes chez des patients atteints du SIDA et donc de prolonger leur vie (Bouic, P.J.D. AIDS Bulletin, v. 6 #3, p 18-20, Sept. 1997), et,

- un effet anti-diabétique, anti-hyperglycémique (M.D. Ivorra et al: Archives of the International Pharmacodyn, v. 296, pp. 224-231, April 1988) ainsi qu'un effet anti-ulcère, anti-inflammatoire et anti-pyrétique (M.B. Gupta et al: Planta medica (Journal of Medicinal Plant Research) vol. 39, p 157-163, 1980).

Les extraits de l'invention, de par leur composition, présentent donc un large spectre d'activité et sont utilisables dans de nombreux domaines d'applications.

On citera, en particulier, l'utilisation des extraits de l'invention dans le domaine alimentaire. Ces extraits constituent en effet des additifs à forte valeur ajoutée.

Ils conviennent, en particulier, à la supplémentation, par exemple, de chocolats, boissons, produits laitiers.

On citera ainsi l'utilisation des extraits selon l'invention comme additifs à certains aliments pour en faire de véritables aliments-santé (aussi appelés aliments fonctionnels ou alicaments) pour lesquels les allégations santé découleraient de l'ensemble des propriétés biologiques qu'on leur connaît.

L'invention vise aussi à protéger les compléments nutritionnels renfermant une quantité efficace d'extraits selon l'invention.

En particulier, les compléments nutritionnels selon l'invention comprennent au moins un extrait selon l'invention à raison de 25 à 300 mg, de préférence de 100 à 200 mg.

Leur administration par voie orale, sous forme de comprimés, capsules, gélules est particulièrement adaptée.

Les extraits de l'invention présentent également un intérêt tout particulier dans le domaine cosmétique, où leurs propriétés sont avantageusement mises à profit pour entrer dans la composition de formulations, en tant que principes actifs, ou en combinaison avec d'autres principes actifs.

De telles compositions cosmétiques sont donc caractérisées en ce qu'elles renferment une quantité efficace, pour une application cosmétologique, des extraits de l'invention, en association avec les véhicules classiquement utilisés en cosmétologie. Ces extraits seront ainsi utilisés dans l'élaboration de crèmes, lotions, mousses, savons et autres.

Les propriétés des extraits de l'invention leur confèrent également un grand intérêt en thérapeutique. Comme indiqué plus haut, l'étude des propriétés pharmacologiques de leurs constituants a montré leur efficacité dans diverses affections. Ces propriétés s'accompagnent, en outre, d'une grande innocuité de ces produits, qui présentent donc un indice thérapeutique particulièrement satisfaisant.

L'invention vise ainsi l'utilisation de l'extrait selon l'invention comme source de choix pour l'obtention de principes actifs destinés à une utilisation pharmaceutique.

D'autres caractéristiques et avantages de la présente invention ressortiront de la description faite ci-après en référence aux dessins annexés qui en illustrent deux exemples de mise en oeuvre dépourvus de tout caractère limitatif. Sur les dessins :

- la figure 1 est un schéma représentant les étapes successives du procédé, objet de l'invention, et,
- la figure 2 est un schéma similaire à celui de la figure 1 illustrant la variante du procédé de l'invention selon laquelle on met en oeuvre des fèves marchandes dont les grains sont réhumidifiés avant de subir les opérations de concassage dans un solvant d'extraction et de distillation.

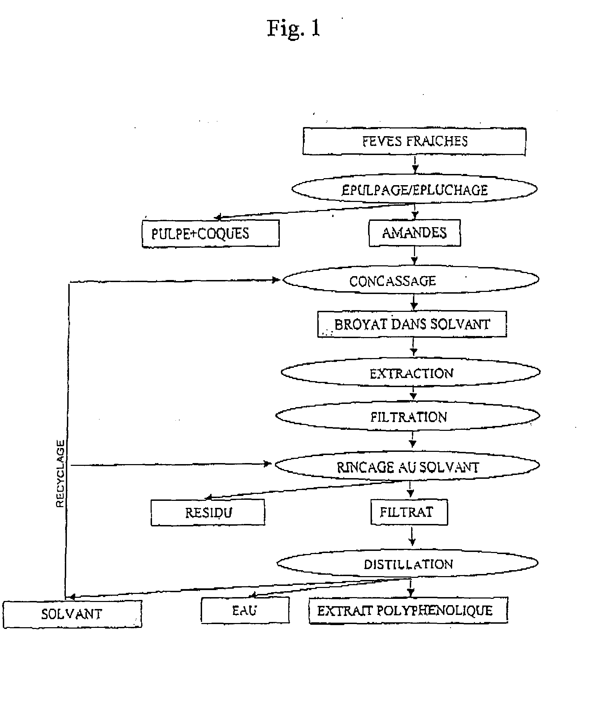

On se réfère en premier lieu à la figure 1.

Selon l'invention, les fèves fraîches, pré-traitées ou non, sont débarrassées de la pulpe et de la coque par une opération d'épulpage/épluchage, par exemple en utilisant un dispositif du type « parmentière ». On obtient ainsi des amandes propres qui sont concassées, par exemple dans un broyeur à couteaux, en présence d'un solvant. Comme précisé ci-dessus, ce solvant est avantageusement choisi dans le groupe comprenant notamment l'eau, l'éthanol, l'acétone, le butanol 2, le propanol 2, en toutes proportions, en mélange avec de l'eau. De préférence, la teneur en solvant est supérieure à 50% en volume (en tenant compte de l'eau apportée par les fèves).

Le mélange amandes concassées/solvant peut être laissé à infuser de quelques heures à plusieurs jours à chaud ou à froid. Si cette infusion est effectuée à chaud, il convient d'éviter les températures trop élevées (c'est-à-dire supérieures à 60°C), afin de limiter l'oxydation chimique et la dégradation chimique des composés à extraire.

Le mélange est ensuite filtré et rincé plusieurs fois à l'aide du solvant utilisé.

On effectue ensuite une distillation en vue d'obtenir un extrait.

Cette distillation est de préférence conduite à une température de 50 à 60°C afin d'éviter une dégradation des composés polyphéniques, sous une pression résiduelle de 12 à 20 KPa, en vue d'évaporer le mélange de solvants contenus dans le filtrat.

Comme indiqué sur le schéma de la figure 1, le solvant récupéré lors de l'étape de distillation du filtrat peut être recyclé dans l'étape de concassage des amandes.

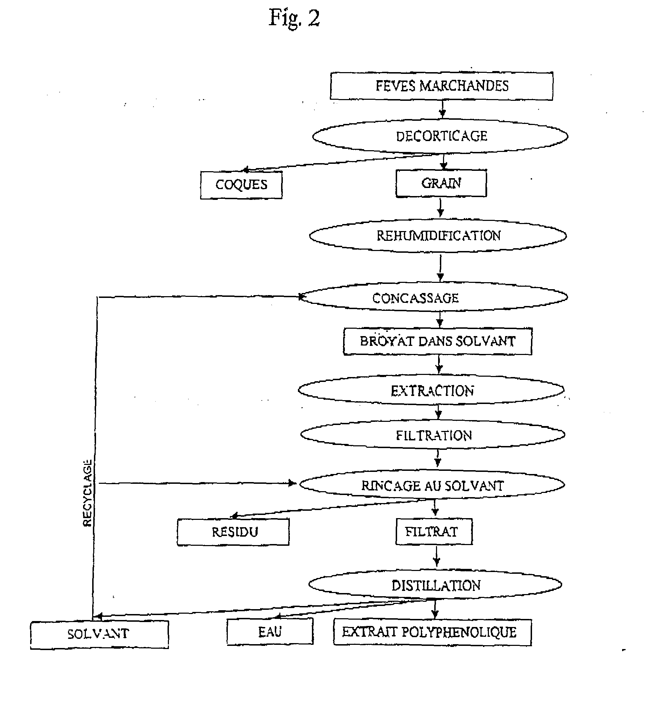

La variante du procédé illustrée par la figure 2 diffère du procédé décrit ci-dessus en référence à la figure 1 uniquement par le fait que le procédé est mis en oeuvre à partir de fèves marchandes de cacao, c'est-à-dire de fèves ayant subi un pré-traitement comportant un séchage, les grains ainsi obtenus étant ensuite soumis à une étape de réhumidification après décorticage, cette étape étant réalisée avec 30 à 50% d'eau tiède, avant l'étape de concassage dans le solvant. Une telle réhumidification permet aux parois cellulaires des amandes de retrouver leur élasticité et donc de ne pas être brisées lors du concassage en présence du solvant. Le pourcentage de lipides extraits est dans ce cas plus important qu'avec les fèves fraîches non réhumidifiées.

On donne ci-après des exemples d'extraits résultants de mises en oeuvre du procédé objet de l'invention.

Dans ces exemples, les xanthines représentent la théobromine et la caféine,

On notera que les pourcentages en polyphénols sont exprimés en équivalent acide gallique, selon la méthode FOLIN CIOCALTEU.

L'activité anti-radicalaire a été évaluée selon le test au DPPH (radical 1,1 diphenyl-2-picryl-hydroxy). Il est nécessaire de connaître la concentration molaire des solutions d'extraits soumis au test. Or, la nature de toutes les molécules présentes n'étant pas connue, une « estimation » d'un poids moléculaire moyen (celui de la catéchine) est alors choisie (arbitrairement) pour exprimer cette molarité. Les résultats sont alors exprimés par le nombre de micromoles nécessaires pour réduire 50% des formes radicalaires du DPPH. D'où, plus la valeur est élevée, moins l'extrait est anti-radicalaire.

Le pourcentage d'extrait/sec (extrait exprimé sur la matière sèche) est déterminé à l'aide de la relation suivante :

$$\% \text{ Extrait/sec} = \frac{10\ 000 * E}{W * (100-H)}$$

où :

- E désigne le poids de l'extrait en grammes,
- W désigne le poids des fèves en grammes, et,
- H désigne le taux d'humidité des fèves.

Cette relation permet de rendre comparable les résultats obtenus sur des fèves d'origine différentes. En effet, le taux d'humidité d'une fève est variable selon son origine.

Le dosage des xanthines a été effectué selon la méthode OICCC n°107 (1988).

La composition de la matière grasse a été déterminée d'après la méthode de C.C. Young (voir ″The interprétation of GLC Triglycérides Data for the Determination of Cocoa Butter Equivalents in chocolate. A new approach." 1984, JAOCS, 61, p 576-581). L'exploitation des résultats selon PADLEY permet d'interpréter la composition de la partie lipidique (acide gras, stérols, triglycérides...) de l'extrait.

**1) Extraction selon l'invention :**

Variation des rendements d'extraction en fonction du type de solvant (24h de macération)

| | Eau froide | MeOH (70%) | Eau à 60°C | Acétone (70%) | Propanol 2 (70%) | Ethanol (70%) | Butanol 2 (70%) |
|---|---|---|---|---|---|---|---|
| **%extrait/sec** | 8,8 | 10,6 | 13,4 | 12 | 14,4 | 11,1 | 10,1 |
| **Composition extrait (%)** | | | | | | | |
| Lipides | 0,6 | 0 | 8,7 | 0,1 | 3,6 | 3,5 | 10,4 |
| Xanthines | 8,6 | 6 | 9,4 | 5,6 | 9,9 | 8,5 | 16,1 |
| Polyphénols | 17,2 | 41,2 | 19,1 | 61,1 | 45,5 | 54,3 | 37,6 |
| | | | | | | | |
| Activité DPPH | 88,5 | 36,4 | 147,6 | 25,9 | 39 | 31,2 | 41,6 |

L'extrait renfermant la fraction lipidique la plus intéressante associée à la meilleure activité anti-radicalaire (due à la fraction polyphénolique) est obtenue par le mélange de solvant éthanol - eau (70:30 en volume).

Ce solvant est donc choisi de manière préférentielle,

**2) Comparaison sur les extractions de fèves d'origine de Côte d'ivoire et du Cameroun**

La comparaison est effectuée sur la teneur en stérols de la matière grasse contenue dans les extraits de l'invention.

On rappelle que la composition de la matière grasse de la fève fraîche est peu variable d'une origine à l'autre. Le pourcentage de stérols dans le beurre de cacao est de 0,14 à 0,16%, tandis que le pourcentage de β- sitostérol est de 0,08 à 0,1%.

Dans le tableau ci-dessous, les extractions sont effectuées sur des fèves du Cameroun avec de l'éthanol à 70%.

| | 1H | 2H | 4H | 16H | 2j |
|---|---|---|---|---|---|
| % Acides gras | 1,72 | 1,24 | 1,10 | 1,58 | 2,67 |
| % Stérols totaux | 15,59 | 20,44 | 24,80 | 25,16 | 25,60 |
| % β- sitostérol | 9,75 | 12,67 | 14,63 | 14,59 | 14,87 |
| % Diglycérides | 79,91 | 75,65 | 63,12 | 72,20 | 68,73 |
| % Triglycérides | 2,76 | 2,65 | 2,11 | 1,04 | 2,98 |

Dans le tableau ci-dessous, les extractions sont effectuées sur des fèves de Côte d'Ivoire avec de l'éthanol à 70%.

|  | 1H | 2H | 4H | 16H | 2j |
|---|---|---|---|---|---|
| % Acides gras | 11,79 | 15,30 | 14,47 | 18,02 | 17,62 |
| % Stérols totaux | 19,82 | 17,68 | 20,30 | 20,74 | 20,97 |
| % β-sitostérol | 11,54 | 10,51 | 11,95 | 12,18 | 12,18 |
| % Diglycérides | 62,03 | 61,41 | 59,80 | 55,42 | 56,25 |
| % Triglycérides | 3,86 | 2,00 | 1,70 | 0,99 | 1,20 |

On remarquera que le procédé selon l'invention permet d'enrichir la fraction lipidique en stérols et, en particulier, en β-sitostérol, par rapport au beurre de cacao.

**3) Comparaison des extractions selon l'origine des fèves de cacao (24 heures de macération)**

Extraction à l'éthanol 70%.

|  | Cameroun | Guinée EQ | Côte d'ivoire | Brésil |
|---|---|---|---|---|
| **% extrait/sec** | 11,1 | 9,5 | 8,4 | 12 |
| **Composition extrait %** | | | | |
| Lipides | 3,5 | 4,9 | 9,2 | 3,4 |
| Xanthines | 8,5 | 8,3 | 10,4 | 14,2 |
| Polyphénols | 54,3 | 40,2 | 47,6 | 38,2 |
|  | | | | |
| Activité DPPH | 31,2 | 42 | 47,9 | 40,6 |

**4) Extraction sur fèves marchandes (24 heures de macération)**

Extraction à l'éthanol 70%.

|  | Côte d'Ivoire |
|---|---|
| **% extrait/sec** | 9,4 |
| **Composition extrait (%)** | |
| Lipides | 9,5 |
| Xanthines | 10,5 |
| Polyphénols | 19 |
|  | |

7

(suite)

| Composition extrait (%) | |
|---|---|
| Activité anti-radicalaire | 99 |

## 5) Composition dans le domaine agro-alimentaire

On a indiqué ci-après des exemples non limitatifs d'utilisation d'extraits selon l'invention, obtenus par la mise en oeuvre du procédé défini ci-dessus. Ces extraits peuvent être utilisés en temps que supplémentation dans de nombreux produits alimentaires. Le titulaire a testé l'addition d'extraits obtenus avec de l'éthanol à 70%, dans des produits chocolatés. Dans tous les exemples indiqués ci-après, on a procédé à une comparaison entre le produit avec addition d'extraits et un produit exempt d'une telle addition :

CHOCOLAT NOIR

| Composition : pâte de cacao : | 56% |
|---|---|
| Sucre | 26,99% |
| Beurre de cacao | 16% |
| Vanille | 0,01% |
| Extrait de l'invention: | 1% |

Dégustation : le jury était composé de 18 personnes : 12 personnes sur 18 ont préféré le chocolat avec addition d'extrait selon l'invention. Il a été trouvé plus rond et plus aromatique.

CHOCOLAT AU LAIT

| Composition : pâte de cacao : | 7,60% |
|---|---|
| Sucre | 41,30% |
| Beurre de cacao | 27,50% |
| Lait gras : | 22,50% |
| Lécithine | 0,59% |
| Vanilline : | 0,01% |
| Extrait de l'invention | 0,50% |

Dégustation : le jury était composé de 15 personnes.
Résultat : couleur légèrement plus rosée; aucune différence significative n'a été relevée en ce qui concerne le goût du produit.

PREPARATION DE BOISSON POUR DISTRIBUTEUR AUTOMATIQUE

| Composition sucre : | 52,69% |
|---|---|
| Poudre de cacao fortement dégraissée | 14% |
| Vaniline | 0,01 % |
| Lait (0% M.G.) | 33% |
| Extrait de l'invention | 0,30% |

Dégustation : le jury était composé de 9 personnes.
25 grammes de cette préparation ont été additionnés de 200 ml d'eau chaude.
Résultat : aucune différence significative n'a été relevée entre les deux préparations.
Il demeure bien entendu que la présente invention n'est pas limitée aux divers exemples de mise en oeuvre mentionnés ci-dessus mais qu'elle en englobe toutes les variantes.

**6) Composition dans le domaine cosmétique**

Préparation cosmétique anti-solaire pour lutter contre le vieillissement cutané et amincissante.

On réalise une émulsion E/H en mélangeant un filtre solaire avec l'extrait polyphénolique de cacao selon l'invention et des excipients pour crème. Ce sérum associe des propriétés anti-solaire (par la présence d'un filtre solaire et des polyphénols de l'extrait selon l'invention), anti-rides (par la présence des polyphénols de l'extrait selon l'invention) et amincissantes (par la présence des bases xanthiques de l'extrait selon l'invention).

Formulation :

| | |
|---|---|
| Isopropylméthoxycinnamate et éthyldiisopropylcinnamate (Néo Héliopan E 1000®) | 3 % |
| Extrait de fève de cacao selon l'invention | 3 % |
| Excipients pour sérum E/H | Q.S. |

Composition des excipients :

| | |
|---|---|
| - Propylène glycol dicaprylate/dicarate + stearalkonium hectorite + propylène carbonate (Miglyol 840 gel B®) | 20,0% |
| - Bis-diglycéryl caprylate/caprate/isostéarate/hydroxystéarate adipate (softisan 649®) | 5,0% |
| - Isostéaryl diglycéryl succinate (Imwitor 780 K®) | 5,0% |
| - Huile de paraffine | 8,0 % |
| - Paraffine solide | 3,0% |
| - Sulfate de magnésium | 2,0% |
| - Eau q.s. | 100, 0 % |

**Revendications**

1.  Procédé d'obtention d'extraits à base de composés polyphénoliques contenus dans le cacao, **caractérisé en ce qu'**il comprend :

    - la mise en oeuvre de fèves fraîches, n'ayant pas subi de pré-traitement, ni de dégraissage, ces fèves étant débarrassées de leurs pulpe et coque, de manière à obtenir des amandes propres,
    - le concassage desdites amandes, en présence de solvant(s),
    - la macération des amandes concassées dans des conditions permettant d'extraire les composés recherchés,
    - la filtration du mélange de macération,
    - la récupération d'un extrait renfermant lesdits composés à partir du filtrat

2.  Procédé selon la revendication 1, **caractérisé en ce qu'**on met en oeuvre des fèves de cacao marchandes, c'est-à-dire des fèves ayant subi un pré-traitement comportant un séchage, dont les grains sont réhumidifiés avant concassage par exemple avec 30 à 50% d'eau tiède.

3.  Procédé selon la revendication 1 ou 2, **caractérisé en ce que** l'étape de macération est réalisée avec un ou plusieurs solvants capables de solubiliser les polyphénols et les lipides sans altérer leurs propriétés.

4.  Procédé selon la revendication 3, **caractérisé en ce qu'**on utilise de l'eau ou un mélange d'eau et de solvant, le solvant étant choisi dans le groupe comprenant l'éthanol, l'acétone, le butanol 2, et le propanol 2.

5.  Procédé selon la revendication 3 ou 4, **caractérisé en ce qu'**on utilise un mélange solvant/eau avec une teneur en solvant supérieure à 50% en volume.

6.  Procédé selon l'une quelconque des revendications 1 à 5, **caractérisé en ce que** l'étape de macération est réalisée durant 1 h à plusieurs jours, à chaud ou à froid.

7.  Procédé selon l'une quelconque des revendications 1 à 6, **caractérisé en ce que** la récupération de l'extrait est réalisée par distillation du filtrat obtenu, à une température comprise entre 50 et 60°C, sous une pression résiduelle de 12 à-20 KPa.

8.  Extraits à base de composés polyphénoliques, **caractérisés par** une teneur (% en poids par rapport à l'extrait total) de 15 à 65% en polyphénols, de 0 à 11% en lipides et de 5 à 20% en xanthines.

9.  Extraits selon la revendication 8, **caractérisés en ce que** les lipides comportent de 10 à 30% en poids de phystostérols avec de préférence, 7 à 15% de β-sitostérol.

10. Utilisation des extraits selon la revendication 8 ou 9, dans le domaine alimentaire, en tant qu'additifs, notamment pour la supplémentation de chocolats, baissons, produits laitiers.

11. Utilisation des extraits selon la revendication 8 ou 9, dans le domaine alimentaire, pour la fabrication d'aliments fonctionnels,

12. Compléments nutritionnels **caractérisés en ce qu'**ils renferment une quantité efficace d'extraits selon la revendication 8 ou 9.

13. Compléments nutritionnels selon la revendication 12, **caractérisés en ce qu'**ils comprennent au moins un extrait selon la revendication 8 ou 9, à raison de 25 à 300 mg, de préférence de 100 à 200 mg.

14. Compléments nutritionnels selon la revendication 12 ou 13, **caractérisés en ce qu'**ils sont administrables par voie orale, en particulier sous forme de comprimés, capsules, gélules.

15. Utilisation des extraits selon la revendication 8 ou 9, dans le domaine cosmétique, en tant que principes actifs ou en combinaison avec d'autres principes actifs.

16. Compositions cosmétiques, **caractérisées en ce qu'**elles renferment une quantité efficace, pour une application cosmétologique, des extraits selon la revendication 8 ou 9, en association avec les véhicules classiquement utilisés en cosmétologie.

17. Compositions cosmétiques selon la revendication 16, **caractérisées en ce qu'**elles se présentent sous forme de crèmes, lotions, mousses, savons.

18. Utilisation de l'extrait selon la revendication 8 ou 9, comme source pour l'obtention de principes actifs dans le domaine pharmaceutique.

## Fig. 1

# Fig. 2

```
                                    ┌─────────────────────┐
                                    │  FEVES MARCHANDES   │
                                    └──────────┬──────────┘
                                               ▼
                              ╭────────────────────────────────╮
                              │          DECORTICAGE           │
                              ╰────────────────────────────────╯
                                               │
        ┌──────────┐                           ▼
        │  COQUES  │◄─────────────┐  ┌─────────────────────┐
        └──────────┘              │  │        GRAIN        │
                                  │  └──────────┬──────────┘
                                               ▼
                              ╭────────────────────────────────╮
                              │        REHUMIDIFICATION        │
                              ╰────────────────────────────────╯
                                               │
                                               ▼
                              ╭────────────────────────────────╮
                              │          CONCASSAGE            │
                              ╰────────────────────────────────╯
                                               │
                                               ▼
                              ┌────────────────────────────────┐
                              │      BROYAT DANS SOLVANT       │
                              └────────────────────────────────┘
                                               │
                                               ▼
                              ╭────────────────────────────────╮
                              │          EXTRACTION            │
                              ╰────────────────────────────────╯
                                               │
                                               ▼
                              ╭────────────────────────────────╮
                              │          FILTRATION           │
                              ╰────────────────────────────────╯
                                               │
                                               ▼
                              ╭────────────────────────────────╮
                              │      RINCAGE AU SOLVANT       │
                              ╰────────────────────────────────╯
                                               │
                    ┌──────────────┐           ▼
                    │    RESIDU    │   ┌─────────────────────┐
                    └──────────────┘   │       FILTRAT       │
                                       └──────────┬──────────┘
                                                  ▼
                              ╭────────────────────────────────╮
                              │          DISTILLATION          │
                              ╰────────────────────────────────╯
                                               │
    ┌──────────────┐   ┌──────────────┐        ▼
    │   SOLVANT    │   │     EAU      │  ┌─────────────────────────────┐
    └──────────────┘   └──────────────┘  │    EXTRAIT POLYPHENOLIQUE   │
                                         └─────────────────────────────┘

    RECYCLAGE
```

**Office européen des brevets**

**RAPPORT DE RECHERCHE EUROPEENNE**

Numéro de la demande

EP 07 00 4829

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 0 906 761 A (ARCHER DANIELS MIDLAND CO) 7 avril 1999 (1999-04-07) * page 3, alinéa 18-26; revendications 9,24 * | 1,3-6,8, 15-17 | INV. C07C37/74 A23G1/00 A23G1/04 A23L3/3472 A23L1/30 C07C39/00 |
| X | PATENT ABSTRACTS OF JAPAN vol. 1998, no. 01, 30 janvier 1998 (1998-01-30) & JP 09 234018 A (MEIJI SEIKA KAISHA LTD), 9 septembre 1997 (1997-09-09) * abrégé * | 1,3-6, 10-12, 14,18 | |
| X | PATENT ABSTRACTS OF JAPAN vol. 2000, no. 02, 29 février 2000 (2000-02-29) & JP 11 308978 A (MEIJI SEIKA KAISHA LTD), 9 novembre 1999 (1999-11-09) * abrégé * | 1,3-6,8, 10-12, 14,18 | |
| X | PATENT ABSTRACTS OF JAPAN vol. 1998, no. 01, 30 janvier 1998 (1998-01-30) & JP 09 224606 A (MEIJI SEIKA KAISHA LTD), 2 septembre 1997 (1997-09-02) * abrégé * | 1,3-5,8, 10-12, 14,18 | DOMAINES TECHNIQUES RECHERCHES (IPC)  A23G |
| A | US 4 352 746 A (REHACEK JOSEF ET AL) 5 octobre 1982 (1982-10-05) * colonne 2, ligne 16 - ligne 23 * * colonne 5, ligne 8 - ligne 18; revendications 1,5,6 * | 1,4,5 | |
| A | PATENT ABSTRACTS OF JAPAN vol. 1996, no. 02, 29 février 1996 (1996-02-29) & JP 07 274894 A (MEIJI SEIKA KAISHA LTD), 24 octobre 1995 (1995-10-24) * abrégé * | 1,3,4,8, 10-12, 14,18 | |

-/--

Le présent rapport a été établi pour toutes les revendications

| Lieu de la recherche | Date d'achèvement de la recherche | Examinateur |
|---|---|---|
| Munich | 4 avril 2007 | Breimaier, Waltraud |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**EP 1 787 970 A1**

**Office européen des brevets**

**RAPPORT DE RECHERCHE EUROPEENNE**

Numéro de la demande

EP 07 00 4829

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | PATENT ABSTRACTS OF JAPAN vol. 1995, no. 11, 26 décembre 1995 (1995-12-26) & JP 07 213251 A (MEIJI SEIKA KAISHA LTD), 15 août 1995 (1995-08-15) * abrégé * | 1,3,4,8, 10-12, 14,18 | |
| A | PATENT ABSTRACTS OF JAPAN vol. 018, no. 427 (C-1235), 10 août 1994 (1994-08-10) & JP 06 128164 A (HITOSHI ITO;OTHERS: 01), 10 mai 1994 (1994-05-10) | | |
| X | EP 0 943 675 A (FRISCHE GMBH) 22 septembre 1999 (1999-09-22) * alinéas [0002], [0003]; revendications 1,2 * | 1,2 | |
| A | GB 2 223 944 A (LOTTE CONFECTIONERY CO LTD) 25 avril 1990 (1990-04-25) * revendications; exemples 1,2 * | 1-7 | |
| A | US 6 015 913 A (HAMMERSTONE JR JOHN F ET AL) 18 janvier 2000 (2000-01-18) | | |
| A | WO 99/45788 A (MARS INC ;MYERS MARY E (US); WHITACRE ERIC J (US); NWOSU CHIGOZIE) 16 septembre 1999 (1999-09-16) * page 9, ligne 29 - ligne 32 * * page 13, ligne 30 - page 14, ligne 20; revendications 1,5,7,10,17,25,28 * | 1,8,10, 11 | |
| A | WO 97/36597 A (HAMMERSTONE JOHN F JR ;POST LAURIE S (US); BUCK MARGARET M (US); M) 9 octobre 1997 (1997-10-09) | | |
| A | EP 1 026 164 A (ADM COCOA B V) 9 août 2000 (2000-08-09) | | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| Munich | 4 avril 2007 | Breimaier, Waltraud |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 07 00 4829

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-04-2007

| Document brevet cité au rapport de recherche | | Date de publication | Membre(s) de la famille de brevet(s) | | Date de publication |
|---|---|---|---|---|---|
| EP 0906761 | A | 07-04-1999 | AT | 270894 T | 15-07-2004 |
| | | | AU | 748832 B2 | 13-06-2002 |
| | | | AU | 8787998 A | 22-04-1999 |
| | | | CA | 2249501 A1 | 02-04-1999 |
| | | | DE | 69825003 D1 | 19-08-2004 |
| | | | DE | 69825003 T2 | 11-11-2004 |
| | | | ES | 2224337 T3 | 01-03-2005 |
| | | | HK | 1016879 A1 | 22-04-2005 |
| | | | JP | 11221048 A | 17-08-1999 |
| | | | NO | 984591 A | 06-04-1999 |
| | | | NZ | 332131 A | 29-06-2001 |
| JP 09234018 | A | 09-09-1997 | AUCUN | | |
| JP 11308978 | A | 09-11-1999 | AUCUN | | |
| JP 09224606 | A | 02-09-1997 | AUCUN | | |
| US 4352746 | A | 05-10-1982 | AR | 219624 A1 | 29-08-1980 |
| | | | AU | 531025 B2 | 04-08-1983 |
| | | | AU | 5105879 A | 03-04-1980 |
| | | | CA | 1126498 A1 | 29-06-1982 |
| | | | CH | 634726 A5 | 28-02-1983 |
| | | | DE | 2967252 D1 | 15-11-1984 |
| | | | DK | 381579 A | 30-03-1980 |
| | | | EP | 0009661 A1 | 16-04-1980 |
| | | | EP | 0092680 A1 | 02-11-1983 |
| | | | ES | 8100673 A1 | 01-02-1981 |
| | | | FI | 792824 A | 30-03-1980 |
| | | | GB | 2033768 A | 29-05-1980 |
| | | | GR | 72272 A1 | 11-10-1983 |
| | | | IE | 48949 B1 | 26-06-1985 |
| | | | JP | 1475356 C | 18-01-1989 |
| | | | JP | 55048281 A | 05-04-1980 |
| | | | JP | 63011391 B | 14-03-1988 |
| | | | MY | 9484 A | 31-12-1984 |
| | | | MY | 9584 A | 31-12-1984 |
| | | | NO | 793125 A | 01-04-1980 |
| | | | NZ | 191609 A | 23-01-1981 |
| | | | PT | 70241 A | 01-10-1979 |
| | | | SE | 444499 B | 21-04-1986 |
| | | | SE | 7907579 A | 30-03-1980 |
| | | | SG | 1583 G | 09-09-1983 |
| | | | ZA | 7904914 A | 27-08-1980 |
| JP 07274894 | A | 24-10-1995 | JP | 3063818 B2 | 12-07-2000 |

EPO FORM P0460

Pour tout renseignement concernant cette annexe : voir Journal Officiel de l'Office européen des brevets, No.12/82

**EP 1 787 970 A1**

## ANNEXE AU RAPPORT DE RECHERCHE EUROPEENNE
## RELATIF A LA DEMANDE DE BREVET EUROPEEN NO.

EP 07 00 4829

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-04-2007

| Document brevet cité au rapport de recherche | | Date de publication | Membre(s) de la famille de brevet(s) | | Date de publication |
|---|---|---|---|---|---|
| JP 07213251 | A | 15-08-1995 | JP | 3095605 B2 | 10-10-2000 |
| JP 06128164 | A | 10-05-1994 | AUCUN | | |
| EP 0943675 | A | 22-09-1999 | AT | 242795 T | 15-06-2003 |
| | | | CA | 2264434 A1 | 13-09-1999 |
| | | | DE | 59808691 D1 | 17-07-2003 |
| | | | ES | 2201354 T3 | 16-03-2004 |
| | | | PT | 943675 T | 31-10-2003 |
| | | | US | 6265593 B1 | 24-07-2001 |
| GB 2223944 | A | 25-04-1990 | AUCUN | | |
| US 6015913 | A | 18-01-2000 | AU | 750621 B2 | 25-07-2002 |
| | | | AU | 4336997 A | 26-03-1998 |
| | | | BR | 9713194 A | 31-10-2000 |
| | | | CA | 2264822 A1 | 12-03-1998 |
| | | | CN | 1235518 A | 17-11-1999 |
| | | | EP | 1014804 A1 | 05-07-2000 |
| | | | HK | 1023038 A1 | 24-09-2004 |
| | | | IL | 128848 A | 14-08-2002 |
| | | | JP | 2001500016 T | 09-01-2001 |
| | | | JP | 2003204758 A | 22-07-2003 |
| | | | JP | 2006034291 A | 09-02-2006 |
| | | | RU | 2242880 C2 | 27-12-2004 |
| | | | US | 6194020 B1 | 27-02-2001 |
| | | | WO | 9809533 A1 | 12-03-1998 |
| | | | US | 6372267 B1 | 16-04-2002 |
| WO 9945788 | A | 16-09-1999 | AU | 3082799 A | 27-09-1999 |
| | | | BR | 9908720 A | 08-01-2002 |
| | | | CA | 2323207 A1 | 16-09-1999 |
| | | | CN | 1301133 A | 27-06-2001 |
| | | | EP | 1061812 A1 | 27-12-2000 |
| | | | JP | 2002505860 T | 26-02-2002 |
| | | | PL | 345704 A1 | 02-01-2002 |
| WO 9736597 | A | 09-10-1997 | AU | 5711696 A | 22-10-1997 |
| EP 1026164 | A | 09-08-2000 | AU | 779856 B2 | 17-02-2005 |
| | | | AU | 2977400 A | 25-08-2000 |
| | | | BR | 0007835 A | 10-09-2002 |
| | | | CA | 2361531 A1 | 10-08-2000 |
| | | | JP | 2002536305 T | 29-10-2002 |
| | | | NZ | 513896 A | 28-09-2001 |

Pour tout renseignement concernant cette annexe : voir Journal Officiel de l'Office européen des brevets, No.12/82

EPO FORM P0460

**ANNEXE AU RAPPORT DE RECHERCHE EUROPEENNE
RELATIF A LA DEMANDE DE BREVET EUROPEEN NO.**

EP 07 00 4829

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-04-2007

| Document brevet cité au rapport de recherche | Date de publication | Membre(s) de la famille de brevet(s) | Date de publication |
|---|---|---|---|
| | | WO    0045769  A2 | 10-08-2000 |

Pour tout renseignement concernant cette annexe : voir Journal Officiel de l'Office européen des brevets, No.12/82

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **L.J. PORTER ; Z. MA ; B.G. CHAN.** Cacao procyanidins : major flavanoids and identification of some minor metabolites. *Phytochemistry,* 1991, vol. 35 (5), 1657-1663
- **H. KIM ; P.G. KEENEY.** Epicatechin content in fermented and unfermented cocao beans. *Journal of Food Science,* 1984, vol. 49, 1090-1092
- **PETRONI, A. ; M. BLASEVICH ; M. SALAMI ; N. PAPINI ; G.F. MONTEDORO ; C. GALLI.** Inhibition of platelet aggregation and eicosanoid production by phenolic components of olive oil. *Thromb Res,* 1995, vol. 78 (2), 151-160
- **FRANKEL, E. ; J. KANNER ; J. GERMAN ; E. PARKS ; J. KINSELLA.** Inhibition of oxidation of human low-density lipoprotein by phenolic substances in red wine. *Lancet,* 1993, vol. 341 (8843), 454-457
- **PACE-ASCIAK, C.R. ; S. HAHN ; E.P. DIAMANDIS ; G. SOLEAS ; D.M. GOLDBERG.** The red wine phenolics trans-resveratrol and quercetin block human platelet aggregation and eicosanoid synthesis: implications for protection against coronary heart disease. *Clin Chim Acta,* 1995, vol. 235 (2), 207-219
- **YAMAKOSHI, J. ; S. KATAOKA ; T. KOGA ; T. ARIGA.** Proanthocyanidin-rich extract from grape seeds attenuates the development of aortic atherosclerosis in cholesterol-fed rabbits. *Atherosclerosis,* 1999, vol. 142 (1), 139-149
- **FUHRMAN, B. ; A. LAVY ; M. AVIRAM.** Consumption of red wine with meals reduces the susceptibility of human plasma and low-density lipoprotein to lipid peroxidation. *Am J Clin Nutr,* 1995, vol. 61 (3), 549-554

- **ORGOGOZO, J.M. ; J.F. DARTIGUES ; S. LAFONT ; L. LETENNEUR ; D. COMMENGES ; R. SALAMON ; S. RENAUD ; M. BRETELER.** Wine consumption and dementia in the elderly: A prospective community study in the Bordeaux area. *Rev Neurol,* 1997, vol. 153 (3), 185-192
- **JANG, M.S. ; E.N. CAI ; G.O. UDEANI ; K.V. SLOWING ; C.F. THOMAS ; C.W.W. BEECHER ; H.H.S. FONG ; N.R. FARNSWORTH ; A.D. KINGHORN ; R.G. MEHTA.** Cancer chemopreventive activity of resveratrol, a natural product derived from grapes. *Science,* 1997, vol. 275 (5297), 218-220
- **BOUIC, P.J.D. et al.** *International Journal of Immunopharmacology,* Décembre 1996, vol. 18 (12), 693-700
- *Métab. Clin. Exper.,* 1989, vol. 38, 136-40
- *American J. Clin. Nutr.,* 1994, vol. 59, 1325-31
- **P.J.D. BOUIC et al.** *International Journal of Sports Medicine,* 1999
- **KLIPPEL K. F. et al.** *British Journal of Urology,* Septembre 1997, vol. 80 (3), 427-432
- **BOUIC, P.J.D.** *AIDS Bulletin,* Septembre 1997, vol. 6 (3), 18-20
- **M.D. IVORRA et al.** *Archives of the International Pharmacodyn,* Avril 1988, vol. 296, 224-231
- **M.B. GUPTA et al.** *Planta medica (Journal of Medicinal Plant Research,* 1980, vol. 39, 157-163
- The interprétation of GLC Triglycérides Data for the Determination of Cocoa Butter Equivalents in chocolate. A new approach. *JAOCS,* 1984, vol. 61, 576-581